Europäisches Patentamt

**European Patent Office**

**Office européen des brevets**

(11) Numéro de publication: **0 006 387**
**B1**

(12) **FASCICULE DE BREVET EUROPEEN**

(45) Date de publication du fascicule du brevet: **23.02.83**

(51) Int. Cl.³: **C 07 D 239/42**

(21) Numéro de dépôt: **79400393.9**

(22) Date de dépôt: **15.06.79**

(54) **Nouveau procédé amélioré de préparation de l'isopropylamino-2 pyrimidine.**

(30) Priorité: **16.06.78 NZ 187595**

(43) Date de publication de la demande:
**09.01.80 Bulletin 80/1**

(45) Mention de la délivrance du brevet:
**23.02.83 Bulletin 83/8**

(84) Etats contractants désignés:
**AT BE CH DE FR GB IT LU NL SE**

(56) Documents cités:
**Chemical Abstracts, Volume 63, no. 12, 6
décembre 1965 (COLUMBUS, OHIO U.S.A.) D.
J. Brown et al., "The Dimroth rearrangement.
IV. A study of facilitation by electron-
withdrawal. Alkylated 2-Alkylimino-
pyrimidines" 16166 B.
Chemical Abstracts, Volume 67, no. 3, 17 juillet
1967 (COLUMBUS, OHIO U.S.A.) D. J. Brown et
al., "The Dimroth rearrangement, VIII. Rate
enhancement by electron-withdrawal in simple
iminopyrimidines" page 1027, 10969V.**

(73) Titulaire: **Société d'Expansion Scientifique
EXPANSIA Société Anonyme
264, rue du Faubourg Saint-Honoré
F-75008 Paris (FR)**

(72) Inventeur: **Aspisi, Christian
Les Grands Vallons Chemin du Breuil
F-13150 Boulbon (FR)**
Inventeur: **Demosthene, Claude
Route de Théziers
F-30390 Aramon (FR)**

(74) Mandataire: **Lachassagne, Jacques
Boite Postale 07
F-78430 Louveciennes (FR)**

Courier Press, Leamington Spa, England.

# Nouveau procédé amélioré de préparation de l'isopropylamino-2 pyrimidine

La présente invention concerne un nouveau procédé amélioré de préparation de l'isopropylamino-2 pyrimidine, présentant l'avantage d'une réaction s'effectuant en une seule étape et avec un rendement supérieur par rapport aux procédés déjà connus de préparation de l'isopropylamino-2 pyrimidine à partir de l'amino-2 pyrimidine.

Il est déjà connu de préparer l'isopropylamino-2 pyrimidine à partir de chloro-2 pyrimidine et d'isopropylamine; ceci a été décrit par BROWN D. J. et HARPER J. S. J. Chem. Soc. 5542 (1965).

Il est également connu d'utiliser la voie de l'alcoylation réductive pour la préparation des alcoylamino-2 pyrimidines à partir d'amino-2 pyrimidine et d'acide formique; cf. I. A. KAYE et I. C. KOGON Réc. Trav. Chim. *71*, 309, (1952).

Il est enfin connu que l'isopropylamino-2 pryimidine peut être également préparée par alcoylation d'amino-2 pyrimidine à l'aide d'un halogénure alcoyle, ce qui conduit aux alcoyle-2 dihydro-1,2- imino-2 pyrimidines qui, dans des conditions basiques, se transforment en alcoylamino-2 pyrimidines correspondantes par le réarrangement de DIMROTH (BROWN D. J. et PADDON-ROW M. N. J. Chem. Soc. (C) 903 (1967).

Cependant, toutes ces méthodes bien connues conduisent à l'isopropylamino-2 pyrimidine avec des rendements généralement médiocres, inférieurs à 50%.

Selon la présente invention, il a été montré que l'isopropylamino-2 pyrimidine peut être obtenue avec un très bon rendement, en faisant réagir l'amino-2 pyrimidine sur de l'acétone, en présence d'un acide organique carboxylique et d'un excès de borohydrure alcalin. Dans cette réaction, l'acétone est à la fois l'un des réactifs et le solvant; un grand excès d'acétone est donc nécessaire.

La présente invention sera mieux comprise grâce à l'exemple suivant:

Dans un réacteur de 2 litres, muni de moyens d'agitation, de chauffage et de refroidissement, on verse une solution de 9,5 g (0,1 mole) d'amino-2 pyrimidine dans 100 ml d'acétone anhydre et 100 ml d'acide acétique cristallisable; le mélange réactionnel est maintenu à température ambiante ou de préférence, quelques degrés au-dessous de celle-ci.

On ajoute lentement, en agitant et en maintenant à la même température, 10 g (0,26 mole) de borohydrure de sodium et on continue à agiter pendant 5 heures après la fin de cette addition, à température ambiante.

On ajoute alors 150 ml d'une solution ammoniaque aqueuse à 20% — la température étant maintenue à environ 30°C — puis 100 ml de chloroforme et 50 ml d'eau; après avoir arrêté l'agitation, on obtient une solution en deux phases: la phase chloroformique est séparée et la phase aqueuse est lavée deux fois par 100 ml de chloroforme, à chaque fois. Toutes les phases chloroformiques sont réunies, lavées avec une solution ammoniaque aqueuse, puis à l'eau jusqu'à neutralité, et enfin séchées sur sulfate de sodium.

On obtient ainsi 4 g (rendement 60%) d'isopropylamino-2 pyrimidine d'une grande pureté, ce qui est confirmé par l'analyse.

## Revendication

Procédé de préparation de l'isopropylamino-2 pyrimidine consistant à faire réagir, à la température ambiante, l'amino-2 pyrimidine sur de l'acétone en excès, en présence d'un acide organique carboxylique et d'un excès de borohydrure alcalin.

## Patentanspruch

Verfahren zur Herstellung von Isopropylamino-2-pyrimidin, dadurch gekennzeichnet, daß bei Raumtemperatur Amino-2-pyrimidin mit einem Überschuß an Aceton in Gegenwart einer organischen Carbosaüre und eines Überschusses an Alkaliborhydrid umgesetzt wird.

## Claim

Preparation process of 2-isopropylamino pyrimidine consisting in reacting at room temperature, 2-amino pyrimidine on acetone in the presence of an organic carboxylic acid and of an excess of an alkaline borohydride.